# EUROPEAN PATENT APPLICATION

(11) **EP 1 518 559 A1**
(43) Date of publication of application: **30.03.2005**
(21) Application number: 03445105.4
(22) Date of filing: 26.09.2003
(51) Int. Cl.: A61K 49/00, A61K 33/00, A61K 33/16, A61K 31/01, A61K 31/045

(54) **Use of a gas for the manufacture of a monitoring agent for diagnosing passage through the intestinal tract or exocrine function of the pancreas**

(71) Applicant: AGA AB, 181 81 Lidingö (SE)
(72) Inventor: Hahn, Robert, 146 40 Tullinge (SE)
(74) Representative: Larsson, Kjell

(57) **Abstract**

Use of a gas or gas precursor, which is of such a nature that it is detectable via the expired breath from a mammal, for the manufacture of a diagnostically acceptable monitoring agent for diagnosing passage through the gastrointestinal tract or exocrine function of the pancreas in said mammal, including man. A method and a monitoring agent for such diagnosing.

## Description

### Technical field of the invention

The present invention is within the field of diagnosing gastrointestinal passage or exocrine function of the pancreas in a mammal, including man. More specifically it relates to the use of a gas or gas precursor, which is of such a nature that it is detectable via the expired breath from said mammal.

### Background art

Disorders related to the gastrointestinal passage or exocrine function of the pancreas are common, and simple methods for their diagnosis are in demand. Examples of such disorders and present methods for their diagnosis are presented below.

Abnormalities in gastric emptying have been documented in a wide variety of clinical conditions. Patients usually show non-specific symptoms such as intermittent or constant nausea, vomiting, bloating, or post-prandial abdominal pain. At present, diagnosis of gastric emptying abnormalities is made mainly by scintigraphy, which is time-consuming and expensive as well as demanding to patient and staff.

Absence of gastric emptying due to postoperative ileus, i.e. obstruction of the small bowel after an operation because normal peristalsis is lost during hours or days after surgery, stops a patient from eating until normal gastric emptying has been regained. Thus, a fast, easily accessible and non-demanding method for determination of the condition is in demand.

Pyloric stenosis is the narrowing of the lower portion of the stomach (pylorus) that leads into the small intestine. Since the stomach opening becomes blocked, gastric emptying does not occur and food cannot move into the intestine. This condition typically occurs in infants between 2 and 8 weeks of age. In consideration of the sensitivity of such patients, a fast and non-demanding method for diagnosis is desirable.

Conditions related to intestinal peristalsis disorders are common and include irritable colon and Hirschsprung's disease. For the diagnosis of such conditions it is desirable to study the course of time for passage through the gastrointestinal tract. At present such diagnostic methods, e.g. x-ray examination wherein the patient is given contrast fluid orally, are laborious and demanding to patient and staff.

Malfunction of the exocrinic activity of the pancreas, such as in pancreatitis, exists as various types. In acute pancreatitis enzymes normally secreted by the pancreas in an inactive form become activated inside the pancreas and start to digest the pancreatic tissue. In chronic pancreatitis inflammation and fibrosis cause the destruction of functioning glandular tissue in the pancreas. The resulting lack of pancreatic enzymes interferes with the ability to properly digest fat. The production of insulin is also affected, which can lead to diabetes.

Indications of the exocrine function of the pancreas can be obtained by laboratory methods, such as analysis of fatty acid content or trypsin and chemotrypsin content in faeces. Both methods mentioned involve the collection of stools and are thus demanding to the patient and not desirable. Additionally, they are unspecific and difficult to interpret. An analysis of P-amylase in blood may indicate damage to the pancreas. However, the test for P-amylase is not specific. In research the secretin test is used. Secretin is given intravenously and induces exocrine secretion of the pancreas. Duodenal content is then collected through a duodenal catheter placed through the mouth, oesophagus and stomach. Enzymatic activity is then measured together with other parameters. This method is very demanding to the patient, time consuming and not suitable for everyday clinical practise.

US 5,838,008 describes a method of determining a trace gas concentration in a gas sample, e.g. breath, using Fourier Transform Infrared Spectroscopy. One embodiment of the invention is suitable for use in ¹³C-based breath tests for various conditions and disorders. In these tests a small amount of a substrate is labelled with a ¹³C atom, where normally there would have been a ¹²C atom. When the labelled substrate is ingested and metabolised, one of the metabolic products is ¹³CO₂. This rapidly passes into the bloodstream and then into the lungs from where it is exhaled in the breath. As one example, a ¹³C-triolein breath test for the diagnosis and monitoring of fat malabsorption usually due to disease or the pancreas, particularly in patients with cystic fibrosis, is mentioned. In summary, this method relates to the ingestion of a labelled substrate and detection in exhaled air of an endogenously produced labelled gas.

In fact, several documents, e.g. WO 03/017818, WO 02/057738, EP 1 101 499, WO 97/30351, US 4,676,974 and WO 01/31334, describe methods, substrates or devices for breath tests based on the detection in exhaled air of an endogenously produced gas in connection with gastrointestinal or pancreatic disorders.

### Summary of the invention

The present invention is based on the finding that exogenous gases can be administered to the gastrointestinal tract of a mammal, including man; that said gases will be taken up in the circulation of said mammal from the intestines, but not from the ventricle (stomach), and be brought to the lungs; and that said gases can be detected in the expired breath from said mammal. This process can be used for diagnosing passage through the gastrointestinal tract or exocrine function of the pancreas.

The object of the present invention is thus to provide use of a gas or gas precursor, which is of such a nature that it is detectable via the expired breath from a mammal, for the manufacture of a diagnostically acceptable monitoring agent for diagnosing passage through the gastrointestinal tract or exocrine function of the pancreas in said mammal, including man. The invention provides a rapid, simple, cheap and non-demanding way of diagnosing passage through the gastrointestinal tract or exocrine function of the pancreas. The examination lasts for a short period only as the exogenous gas is taken up and expired within a few minutes. No expensive or complicated equipment is needed, but merely a gas detector which is generally cheap and of a straightforward construction. For instance, in the case of nitrous oxide (N₂O) such monitors are available in anaesthesia machines, which monitors may be used as such or easily converted into more accurate monitors. Hence, requirements on staff are modest. Further, the patient will not be subjected to radiation or contrast medium, as in previously known methods, but only to a quickly eliminated gas.

In one aspect of the invention the monitoring agent is for diagnosis of gastric emptying. Disturbed gastric emptying is among other diseases associated with postoperative ileus and pyloric stenosis.

In another aspect of the invention the monitoring agent is for examination of the course of passage through the ventricle, duodenum, ileum and/or colon. Such a general examination is advantageous for diagnosis of several conditions in the gastrointestinal tract.

In another aspect of the invention the monitoring agent is for diagnosis of intestinal peristalsis disorders. Examples of such disorders are irritable colon and Hirschsprung's disease.

In yet another aspect of the invention the monitoring agent is for diagnosis of the exocrine function of the pancreas in the form of pancreatitis.

In one embodiment of the present invention the gas or gas precursor in the monitoring agent is dissolved or dispersed in a diagnostically acceptable liquid, such as saline or Ringer's acetate. The liquid is preferably an ordinary drink such as water. Hence, after ingestion of the liquid, e.g. gastric emptying can be diagnosed by detection of gas absorbed from the duodenum.

In another embodiment of the present invention the gas or gas precursor in the monitoring agent is comprised in a diagnostically acceptable encapsulation. A suitable encapsulation releases the gas in a specific part of the gastrointestinal tract or under specific conditions only. The encapsulation is preferably adapted to release said gas or gas precursor in the ventricle, duodenum, ileum and/or colon. Thus, the course of passage through the ventricle, duodenum, ileum and/or colon can be diagnosed. Alternatively, as said above, the encapsulation is preferably adapted to release said gas or gas precursor under specific conditions. Thus, e.g. by providing an encapsulation that releases its content at alkaline conditions, the exocrine function of the pancreas, which also affects pH in the duodenum, can be diagnosed.

Encapsulations having the above-mentioned properties are well-known in the pharmaceutical field. As an example, polymeric dragées releasing their content at specific acidic or around neutral pH values, for instance in the range from about 4.5 to about 7.5, corresponding to specific sites in the intestine, are commercially available. In general pH is low in the ventricle (about 1.0), increases substantially in the duodenum and gradually decreases further down the intestine.

Preferably the gas or gas precursor in the monitoring agent is adsorbed to a diagnostically acceptable carrier. Being adsorbed to a carrier prevents the gas from escaping the encapsulation by permeation or leakage. A preferred carrier releases the adsorbed gas or gas precursor in contact with water, i.e. after degradation of the encapsulation. Molecular sieves, materials that contains many small cavities interconnected with pores of precisely uniform size, are an example of a suitable carrier.

Although generally the characteristics of the gas or gas precursor can not be specified in exact figures, it should be easy for a person skilled in the art to screen candidates for gases or gas precursors without undue experimental work. Guidance in this respect follows from the following examples of useful gases or gas precursors:
- nitrous oxide (N₂O);
- noble gases, e.g. krypton and xenon;
- lower (generally C₁-C₆) saturated or unsaturated hydrocarbons, e.g. ethane, ethene, propene and acetylene;
- sulphur hexafluoride (SF₆) ;
- acetone;
- fluorinated lower hydrocarbons or hydrocarbon derivatives, e.g. volatile inhalation anaesthetics such as sevoflurane (fluoromethyl-2,2,2-trifluoro-1-(trifluoromethyl)ethyl ether); and
- ethanol.
Nitrous oxide is especially preferable.

The gas or gas precursor is preferably present in the monitoring agent in a range which makes the gas well detectable but does not cause inebriation to the patient, such as for nitrous oxide a volume in the range from about 2 to about 500 ml, more preferably from about 5 to about 100 ml, as measured in the gaseous state. For other gases corresponding or similar volumes are applicable or can easily be determined. Particularly, more potent gases, such as sevoflurane or acetone, are preferably present in smaller volumes. In the case when the gas or gas precursor is dissolved in a diagnostically acceptable liquid, a volume of liquid in the range from about 5 ml to about 2 1 is suitable. Lower volumes (e.g. 5 to 500 ml) constitute a suitable drink for infants and children, whereas larger volumes (e.g. 100 ml to 2 1) are suitable for adults.

The monitoring agent is preferably for oral administration. Oral ingestion is a simple, natural and non-invasive method for administration of an agent to the gastrointestinal tract. However, administration of the monitoring agent via a catheter to the ventricle is also possible.

There is also provided a method for diagnosing passage through the gastrointestinal tract or exocrine function of the pancreas in a mammal, including man, which comprises administering to the gastrointestinal tract of said mammal a gas or gas precursor, which is of such a nature that it is detectable via the expired breath from said mammal, and monitoring the expired breath from said mammal by means of a gas detector to detect the gas therein.

As to preferable embodiments of said method reference is made to variations and embodiments presented in connection with the use described above.

There is also provided a monitoring agent for diagnosing passage through the gastrointestinal tract or exocrine function of the pancreas in a mammal, including man, which comprises a gas or gas precursor, which is of such a nature that it is detectable via the expired breath from said mammal.

Also regarding preferable embodiments of said monitoring agent reference is made to variations and embodiments presented in connection with the use described above.

Other purposes and advantages with the invention should be apparent to a person skilled in the art after having read the description.

### Brief description of the drawing

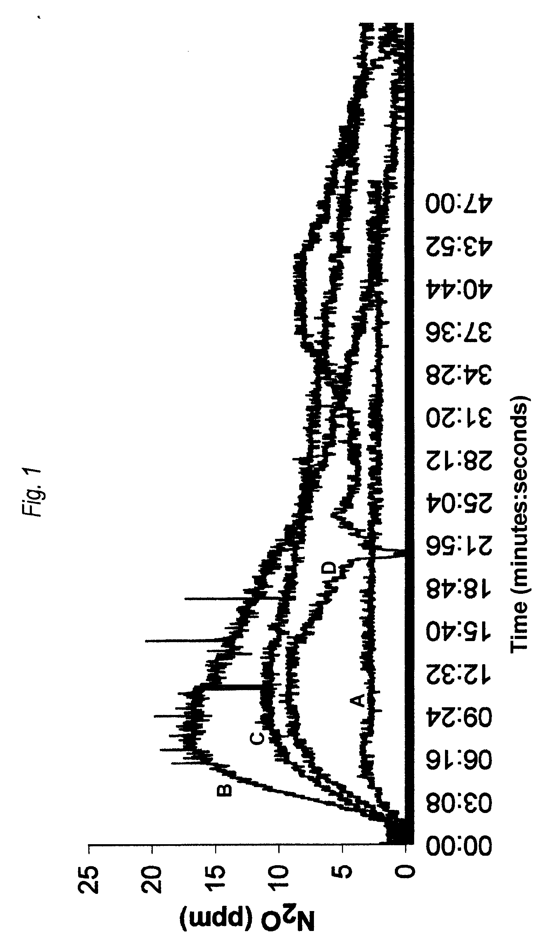

Figure 1 is a graph showing N₂O content versus time in exhaled breath from a pig after injections of 3 ml nitrous oxide (N₂O) dissolved in 30 ml of Ringer's acetate solution to four different parts of the gastrointestinal tract of the pig: the ventricle (A), the duodenum (B), distal ileum (C) and the colon (D). See Example 1.

### Example 1: Uptake of nitrous oxide from different parts of the gastrointestinal tract

A pig was anaesthetised with intravenous ketamin and pethidine and was mechanically ventilated with an oxygen-air mixture. The abdomen was opened surgically and 3 ml nitrous oxide (N₂O) dissolved in 30 ml of Ringer's acetate solution was injected with hourly intervals at four different parts of the gastrointestinal tract: the ventricle, the duodenum, distal ileum and the colon. During injection to the ventricle and the duodenum, the full length of the gastrointestinal tract was open to passage, whereas the injections to distal ileum and the colon were performed after closing off the respective parts of the intestine. The N₂O content versus time in exhaled breath is shown in Figure 1 for uptake from the ventricle (A), the duodenum (B), distal ileum (C) and the colon (D). When injected into the ventricle (A), negligible amounts of N₂O were detected in the exhaled breath of the pig over the subsequent 30 min. This indicates palaytic ileus, i.e. lost peristalsis, whereby no substantial amounts of N₂O pass to the duodenum. After injection to the other locations (B, C, D), however, a marked uptake was noted already after 5 min, reaching a maximum of 20-25 ppm in the exhaled breath. The elimination process was completed after approximately 30 minutes. The pig's hemodynamic situation became impaired in the middle of the final experiment (D), when the dissolved gas had been injected into the pig's colon, with a resulting downward peak in the uptake and subsequent exhalation of N₂O.

### Example 2: Diagnosis of gastric emptying

Gastric emptying relates to the first stage of gastrointestinal passage, i.e. from the ventricle to the duodenum. Thus, uptake in the duodenum of a gas administered to the ventricle indicates working gastric emptying, whereas no uptake of said gas indicates blocking of gastric emptying.

In a preferred way of diagnosing gastric emptying according to the present invention the patient drinks 1 dl of water comprising 10 ml N₂O. Subsequently, N₂O is analysed in expired breath. Presence of N₂O in expired breath indicates uptake in the duodenum of N₂O and hence indicates working gastric emptying. Absence of N₂O in expired breath indicates no uptake of N₂O and hence indicates blocking of gastric emptying.

### Example 3: Preparation of encapsulations

A gas which is detectable in the expired breath of a patient, such as N₂O, is adsorbed on a molecular sieve material that will release the gas upon contact with water. The molecular sieve material with adsorbed gas is encapsulated in a controlled release dragée material. The dragée material is of such a nature that it will be degraded and release the molecular sieve material, and hence the gas, at a specific site in the intestine, such as the ventricle, duodenum, ileum or colon, and/or at specific pH conditions, such as alkaline conditions.

### Example 4: Diagnosis of the course of passage through the ventricle, duodenum, ileum and colon

A way of examining the course of passage through the ventricle, duodenum, ileum and colon according to the invention is to administer to a patient a combination of gas dissolved in a liquid according to Example 2 and/or encapsulations according to Example 3, each of the preparations releasing gas at a different, specific, site in the gastrointestinal tract. By studying the presence of said gases in expired breath over time, the course of passage will be clarified. The gases may be the same, as subsequent peaks in expired breath will be related to subsequent portions of the intestine, but different gases for adjacent portions of the intestine will present the course of passage even clearer.

In a preferred embodiment the following combination is used.

| Gas | Preparation | Released in | Indicates |
|---|---|---|---|
| N₂O | Water | Ventricle | Gastric |
| | | | emptying |
| Sevoflurane | Encapsulation degrading | Duodenum | Passage of |
| | at pH 7.5 | | duodenum |
| N₂O | Encapsulation degrading | Ileum | Passage of |
| | at pH 6 | | ileum |
| Sevoflurane | Encapsulation degrading | Colon | Passage of |
| | at pH 4.5 | | colon |

### Example 5: Diagnosis of exocrine function of the pancreas

Pancreatic juice having a high pH is secreted by the working pancreas into the duodenum. Thus, uptake of a gas released in the duodenum at alkaline conditions only indicates a working pancreas, whereas no release and hence no uptake of said gas indicates a malfunctioning pancreas.

In a preferred way of diagnosing the exocrine function of the pancreas an encapsulation comprising N₂O is formed according to Example 3 using a controlled release dragée material which will be degraded at alkaline conditions. The patient ingests said encapsulation. Subsequently, N₂O is analysed in expired breath. If alkaline conditions exists in the duodenum (working pancreatic secretion), the encapsulation will degrade and the gas be released, taken up and transported to the lungs. Thus, presence of N₂O in expired breath indicates a working pancreas. If alkaline conditions does not exist in the duodenum (malfunctioning pancreatic secretion), the encapsulation will not degrade and no gas will be taken up. Thus, absence of N₂O in expired breath indicates a malfunctioning pancreas.

## Claims

1. Use of a gas or gas precursor, which is of such a nature that it is detectable via the expired breath from a mammal, for the manufacture of a diagnostically acceptable monitoring agent for diagnosing passage through the gastrointestinal tract or exocrine function of the pancreas in said mammal, including man.

2. Use according to claim 1, wherein said passage through the gastrointestinal tract relates to gastric emptying.

3. Use according to claim 2, wherein said gastric emptying relates to postoperative ileus or pyloric stenosis.

4. Use according to any one of claims 1 to 3, wherein said passage through the gastrointestinal tract relates to the course of passage through the ventricle, duodenum, ileum and/or colon of said mammal.

5. Use according to any one of claims 1 to 4, wherein said passage through the gastrointestinal tract relates to an intestinal peristalsis disorder.

6. Use according to claim 5, wherein said intestinal peristalsis disorder is irritable colon or Hirschsprung's disease.

7. Use according to any one of claims 1 to 6, wherein said exocrine function of the pancreas relates to pancreatitis.

8. Use according to any one of claims 1 to 7, wherein said gas or gas precursor in said monitoring agent is dissolved or dispersed in a diagnostically acceptable liquid.

9. Use according to any one of claims 1 to 8, wherein said gas or gas precursor in said monitoring agent is comprised in a diagnostically acceptable encapsulation.

10. Use according to claim 9, wherein said encapsulation is adapted to release said gas or gas precursor in the ventricle, duodenum, ileum and/or colon of said mammal.

11. Use according to claim 9 or 10, wherein said encapsulation is adapted to release said gas or gas precursor at alkaline conditions.

12. Use according to any one of claims 9 to 11, wherein said gas or gas precursor in said monitoring agent is adsorbed to a diagnostically acceptable carrier.

13. Use according to any one of the preceding claims, wherein said gas is selected from nitrous oxide; a noble gas, preferably krypton or xenon; a lower hydrocarbon, preferably ethane, ethene, propene or acetylene; sulphur hexafluoride; and acetone.

14. Use according to claim 13, wherein said gas is nitrous oxide.

15. Use according to claim 14, wherein said nitrous oxide is present in said monitoring agent in a volume in the range from about 2 to about 500 ml, as measured in the gaseous state.

16. Use according to any one of claims 1 to 12, wherein said gas precursor is liquid.

17. Use according to claim 16, wherein said gas precursor is a fluorinated lower hydrocarbon or hydrocarbon derivative, preferably sevoflurane, or ethanol.

18. Use according to any one of the preceding claims, wherein said manufacture is for the manufacture of an orally administrable monitoring agent.

19. A method for diagnosing passage through the gastrointestinal tract or exocrine function of the pancreas in a mammal, including man, which comprises administering to the gastrointestinal tract of said mammal a gas or gas precursor, which is of such a nature that it is detectable via the expired breath from said mammal, and monitoring the expired breath from said mammal by means of a gas detector to detect the gas therein.

20. A method according to claim 19, as defined in any one of claims 2 to 18.

21. A monitoring agent for diagnosing passage through the gastrointestinal tract or exocrine function of the pancreas in a mammal, including man, which comprises a gas or gas precursor, which is of such a nature that it is detectable via the expired breath from said mammal.

22. A monitoring agent according to claim 21, for use as defined in any one of claims 2 to 18.
